# EUROPEAN PATENT APPLICATION

(11) **EP 3 578 063 A1**
(43) Date of publication of application: **11.12.2019**
(21) Application number: 19186316.6
(22) Date of filing: 26.10.2012
(51) Int. Cl.: A24B 15/24, A24F 47/00

(54) **APPARATUS FOR CREATING LIQUID TOBACCO EXTRACT**

(30) Priority: 28.10.2011 GB 201118689
(62) Divisional of application: 12787398.2
(71) Applicant: JT International SA, 1202 Geneva (CH)
(72) Inventor: HOPPS, Jason, Londonderry, BT52 1WR (GB)
(74) Representative: Gill Jennings & Every LLP

(57) **Abstract**

A kit is disclosed for supplying a cartridge of an inhaler article with liquid tobacco extract, the kit comprising an apparatus having means (15) for extracting a liquid tobacco extract from a tobacco derived solid. The apparatus is adapted to deliver liquid tobacco extract to a cartridge (4) and a cartridge adapted to receive liquid tobacco extract from the apparatus. An apparatus for supplying a liquid tobacco extract to a cartridge of an inhaler article is further disclosed. The apparatus has a housing (14), means for extracting (15) a liquid tobacco extract from a tobacco derived solid and said housing is adapted to deliver liquid tobacco extract to a cartridge of an inhaler article. A related method of supplying a cartridge of an inhaler article with a liquid tobacco extract is further disclosed.

## Description

### Field of the Invention

The present invention relates to a method, kit and apparatus for providing the cartridge of an inhaler article with a liquid tobacco extract.

### Background

An inhaler article is a device that simulates the act of tobacco smoking by producing an inhaled mist which closely matches flavor of inhaled tobacco smoke. Inhaler articles can produce inhalable mist by many different mechanisms, for example by heating a propylene glycol or glycerin-based liquid solution and allowing the vapourised solution to condense into microscopic droplets. The heat can be produced by energy supplied by a fuel or by electricity usually stored into a battery. Most inhaler articles are designed to resemble actual tobacco smoking implements, such as cigarettes, cigars, or pipes.

An example of an inhaler that produces an inhalable mist from a battery-based electrical energy is an electronic cigarette. Electronic cigarettes generally consist of three primary components: a cartridge, an atomizer, and a battery unit. The cartridge comprises an absorbent mesh that is soaked with a solution of flavour and aroma compounds, propylene glycol, water and aroma compounds. The cartridge is usually disposable such that when the user senses that the amount of flavour and aroma compounds in the cartridge are run down he is able to replace the used cartridge with a new cartridge.

The inhalable mist generated from such inhalers often lacks specific sensory attributes associated with tobacco smoking implements, since they usually do not contain tobacco. Attempts have been made to overcome this problem by providing solid tobacco within the device as the source of flavour and aroma.

It is desirable to extract the flavour and aroma compounds as close as possible in time to the moment when the user operates the electronic inhaler device, to closely match the flavour of inhaled tobacco smoke, and thus the smoking sensation. Attempts have been made to overcome this problem by providing cut tobacco within the cartridge as the source of flavour and aroma. For example, WO2008/108889 discloses various configurations of an e-cigarette in which a cartridge is loaded. The cartridge may have cut tobacco or tobacco extract. However, because there must be an unimpeded physical flow of liquid from the cartridge to the heating element during use, cut tobacco is not readily suitable for direct insertion into existing cartridge for inhaler articles because the tobacco particles can easily impede the flow of liquid towards the heating element. Furthermore, tobacco particles can easily cause failure of the wick and heating elements of an electronic inhalable article.

An apparatus for extracting a liquid tobacco extract from a tobacco derived solid is described in US2011/0036365.

"How to Refill E-Liq" website www.e-cig.com, 28 March 2010 discloses a refill cartridge for an electronic cigarette.

The present invention aims to address the problems associated with the prior art.

### Summary of the invention

The inventors of the present invention have overcome the problems in the prior art by designing apparatus which may be used by the end user to extract a liquid tobacco extract from a tobacco derived solid and delivering the extract to a refillable cartridge of an inhaler article. As a result, the end user is able to generate the liquid flavour and aroma extract during refilling of the cartridge whilst avoiding the use of a tobacco derived solid within the cartridge.

The tobacco derived solid as referred above can mean cut, ground, reconstituted, shredded, milled, or processed in any way by which its particle size is decreased. In the following described exemplary embodiments, the tobacco derived solid will be cut tobacco.

The present invention is an apparatus for supplying a liquid tobacco extract to a cartridge of an inhaler article, the apparatus comprising a housing having disposed therein a means for extracting a liquid tobacco extract from a tobacco derived solid derived solid wherein said housing is adapted to deliver liquid tobacco extract to a cartridge of an inhaler article, wherein said housing is a deformable bottle having stored therein a mixture of tobacco derived solid and solvent, which bottle dispenses liquid tobacco extract to the cartridge when an external deformable force is applied to the housing.

The present invention is also a kit comprising an apparatus of the above type and a cartridge.

The present invention is also a method of supplying a cartridge of an inhaler article with a liquid tobacco comprising:
a. providing an apparatus of any preceding claim;
b. loading the housing of the apparatus with tobacco derived solid and a solvent;
c. extracting a tobacco extract from the tobacco derived solid to form a liquid tobacco extract and solvent mixture; and
d. providing a cartridge of an inhaler article and delivering the tobacco extract to the cartridge

### Detailed Description of the Invention

The present invention provides apparatus, a kit and a method for providing a liquid tobacco extract from cut tobacco for refilling a cartridge of an inhaler article. Preferably, the inhaler article is an electronic cigarette, cigar or pipe.

The apparatus comprises a housing in which cut tobacco and a solvent is provided. The apparatus may be supplied to the end user with tobacco and solvent already present within the housing. The housing may be adapted such that the user may supply his own cut tobacco in to the apparatus from which he extracts liquid tobacco extract. The cut tobacco may be any tobacco known in the art from which a liquid tobacco extract may be obtained for use in refilling the cartridge of an inhaler article. The solvent may be any solvent used in the art for carrying and impregnating tobacco extract in to a cartridge of an inhaler article. Preferably the solvent is water, propylene glycol, glycerol or mixtures thereof.

The cut tobacco and solvent may be provided as a mixture within the housing. The housing may be adapted to keep the cut tobacco and solvent separate such that the solvent is mixed with the tobacco by the user during the extraction process.

A liquid tobacco extraction means is provided within the housing of the apparatus of the present invention. The extraction means may be a filter which allows the passage of a solvent with tobacco extract through but which retains tobacco particles.

Particles of tobacco-derived solid can be removed from the liquid extract by a variety of means, such as physical separation (for example, steel mesh filtration or a Franz cell silicone membrane). Additionally, chemical filtration can be applied, wherein desirable components of the tobacco-derived solid can freely pass into the liquid extract, and undesirable components of the tobacco-derived solid cannot pass through the filtration mechanism. Thus, the filtration mechanism can be designed according to which elements of the tobacco-derived solid are desirable in the liquid extract, for example, to control intensity of tobacco-like aroma.

Examples of chemical filtration mechanisms include selectively-permeable membranes, such as those used in dialysis or reverse osmosis. The materials used to construct such membranes can control the membranes permeability to various chemical groups, based on key parameters such as molecular size, molecular weight or polarity. Examples of such materials include polyimide, polydimethylsiloxane and regenerated cellulose.

The apparatus of the present invention may be in the form of a bottle which is adapted to detachably receive a cartridge. The bottle may comprise a housing which is deformable such that liquid tobacco extract may be dispensed from the bottle into the cartridge when an external deformable force is applied to the housing by the user. In this embodiment it is preferable that the cut tobacco is provided as a mixture with the solvent. Preferably the apparatus has a housing in the form of a cylinder, such as a hollow pen shaft. A plunger may be slideably mounted within the interior of the shaft which forces solvent to pass through the cut tobacco during the extraction process. More preferably, the apparatus may be in the form of a metered dose spray which delivers a measured dose of liquid tobacco extract to a cartridge.

The housing of the apparatus of the invention may be adapted to detachably receive a cartridge such that liquid tobacco extract may be dispensed from the apparatus into the cartridge and the cartridge then removed from the apparatus for use in the inhaler article. The apparatus may comprise the detachable cartridge or the apparatus may be provided along with the cartridge as a separate component but in a kit of parts. Preferably the apparatus is provided without the cartridge.

Examples of the present invention will now be described with reference to the accompanying drawings, in which:
Figure 1 shows an electronic cigarette and a disposable cartridge.
Figure 2 shows a metered spray bottle for delivering liquid tobacco extract to a disposable cartridge.
Figure 3 shows a dropper bottle for delivering liquid tobacco extract to a disposable cartridge.
Figure 4 shows an apparatus of the invention having a plunger mechanism for extract liquid tobacco extract from cut tobacco.
Figure 5 shows an apparatus of the invention having a detachable cartridge into which liquid tobacco extract may be dispensed from the apparatus.

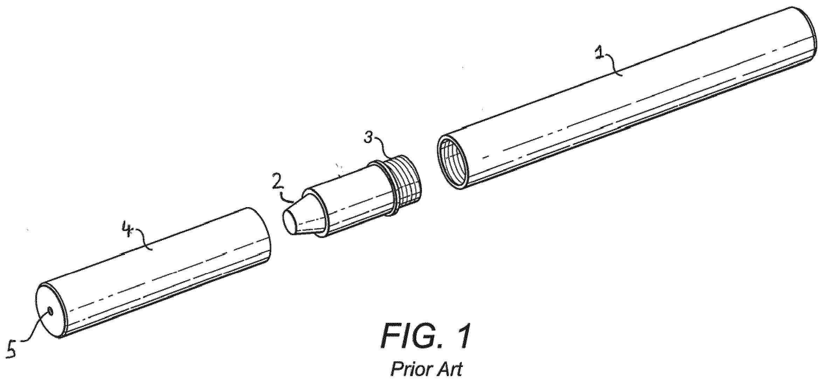

With reference to figure 1 an electronic cigarette has three component parts; a rechargeable battery and circuitry (1), a heater (2) and wick (3) and refillable flavour and aroma containing cartridge (4). The cartridge (4) comprises a housing in which is positioned an absorbent mesh or fibre matrix soaked in a solution of flavour and aroma, propylene glycol, water and aroma compounds. The heater (2) comprising a metal wire wrapped around an insulator is position at the non-smoking end of the cartridge (4) the other end of the cartridge (4) provided with a hole (5) which allows passage of air through the cartridge to the outside during the smoking process. The wick (3) comprises a porous and heart resistant material which is in close proximity to the heat source which is able to transfer volatilised propylene glycol having flavour and aroma compounds therein.

In use, the propylene glycol flows from the matrix towards the heater (2) due to lower pressure during puffing, capillary action and gravity depending on how the electronic cigarette is held. As the propylene glycol is heated and volatilised by the heater (2), general air flow through the cartridge condenses the vapour into small droplets (aerosol), which then pass through the hole (5) and into the user's mouth.

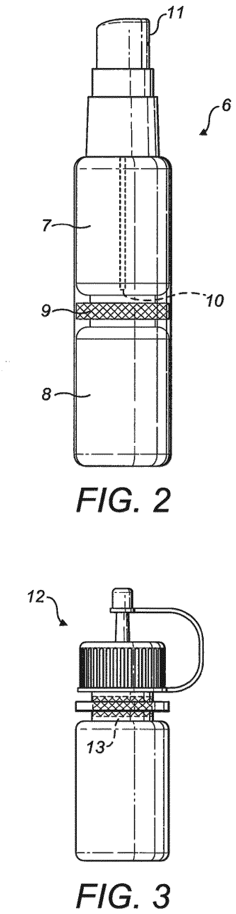

The cartridge (4) may be refilled with liquid tobacco extract using the apparatus shown in Figure 2. In this embodiment, the apparatus is in the form of a metered dose spray bottle (6) which delivers a predetermined amount of liquid tobacco extract to the cartridge (4) per spray. The bottle (6) is divided into an upper (7) and lower (8) compartment by a selectively permeable membrane (9), such as a Franz cell silicone membrane, or a polydimethylsiloxane membrane positioned across the interior lumen of the bottle (6). The lower compartment (8) comprises a mixture of cut tobacco and propylene glycol and the upper compartment (7) comprises propylene glycol only. The free end of a spray tube (10) is position within the propylene glycol of the upper compartment (7) such that actuation of the spray nozzle (11) causes propylene glycol to pass up through the spray tube (10) which causes tobacco solution to diffuse through the membrane and out of the nozzle (11) as a spray into a cartridge. The membrane (9) acts to stop cut tobacco particles from passing from the lower compartment (8). Thus the membrane is adapted to act as a physical barrier to let the liquid pass from the lower to the upper compartment while retaining the tobacco particles, and also a chemical filtration means, whereby the choice and thickness of membrane material can select which tobacco components may pass through, based on for example, polarity or molecular size.

An alternative embodiment is shown in figure 3. A dropper bottle (12) is provided made from a deformable plastic material such as PVC. A steel mesh filter (13) is provided at the neck of the bottle (12). A mixture of cut tobacco and propylene glycol is provided within the bottle such that tobacco extract is able to dissolve in the propylene glycol. During use, the user squeezes the deformable bottle (12) which causes an increase in pressure inside the bottle. Liquid tobacco extract is forced through the steel mesh filter (13) and emerges from the bottle as drops of tobacco extract which are absorbed onto the cartridge. The mesh filter (13) retains the cut tobacco particles such that the particles do not pass into the cartridge.

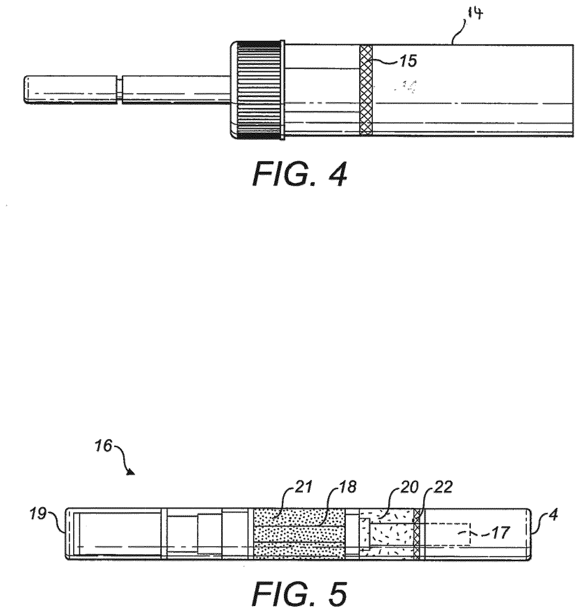

Further alternative embodiments are shown in figures 4 and 5. Figure 4 shows an apparatus having a generally cylindrical housing (14) into which is provided a mixture of cut tobacco and propylene glycol. A plunger (15) having a steel mesh filter is slideably engaged within the internal lumen of the housing (14). In use, the user forces the plunger (15) along the internal lumen of the housing (14) such that liquid tobacco extract passes through the steel mesh filter and cut tobacco particles are pushed towards the bottom of the housing (14). The liquid tobacco extract is then poured from an open end of the housing (14) into a cartridge.

The apparatus shown in the embodiment depicted in figure 5 also employs a plunger type mechanism to separate liquid tobacco extract from cut tobacco particles. The apparatus generally resembles a pen (16) having an open end (17) which is releasably engaged with a cartridge (4). A plunger (18) is engaged in a screw fit with the internal walls of the housing of the pen shaft the plunger having a handle (19) operable by the user. A dry tobacco mix (20) and propylene glycol (21) are provided separately in the internal lumen of the pen shaft between the plunger and opening of the pen. A coarse filter (22) is provided inside the pen shaft immediately before the opening.

In use, the user turns the plunger at the handle (19) which causes the plunger to pass within the lumen of the pen towards the open end (17). As a result, the propylene glycol (21) is forced through the dry cut tobacco (20) and tobacco extract is dissolved into the propylene glycol. Continued turning of the plunger forces the liquid tobacco extract to pass through the coarse filter (22) and out through the opening (17) and into the matrix of the cartridge (4) with which the opening is releasably engaged. Cut tobacco particles (20) are retained by the filter and do not pass through the opening (17).

The apparatus shown in figures 2 to 5 allows the user to make his own liquid tobacco extract from cut tobacco and deliver the extract to a refillable cartridge. Cut tobacco particles are not delivered to the cartridge thereby solving the problems caused by prior art cartridges which use cut tobacco and its effect on cartridge performance.

## Claims

1. An apparatus (6) for supplying a liquid tobacco extract to a cartridge (4) of an inhaler article, the apparatus comprising a housing (6, 12) having disposed therein a means for extracting a liquid tobacco extract from a tobacco derived solid wherein said housing is adapted to deliver liquid tobacco extract to a cartridge (4) of an inhaler article, wherein said means for extracting the liquid tobacco extract is provided as a plunger (18) which is slideably engageable with the external walls of the housing (16).

2. A kit for supplying a cartridge of an inhaler article with liquid tobacco extract, the kit comprising an apparatus according to claim 1, and a cartridge (4) adapted to receive liquid tobacco extract from the apparatus.

3. The kit of claim 2 or apparatus of claim 1, wherein said housing comprises tobacco derived solid and at least one solvent selected from the group consisting of water, propylene glycol, glycerol or mixtures thereof.

4. The kit or apparatus of claim 3, wherein said tobacco derived solid is separate from the solvent or the tobacco derived solid and solvent is provided as a mixture.

5. The kit or apparatus according to any preceding claim, wherein said inhaler article is an electronic cigarette.

6. The apparatus according to any one of claims 1 or 3 to 5, further comprising a cartridge (4) of an inhaler article detachably connected to the housing (6).

7. A method of supplying a cartridge (4) of an inhaler article with a liquid tobacco extract comprising:
a. providing an apparatus of any of claims 1 or 3 to 6;
b. loading the housing (6) of the apparatus with tobacco derived solid and a solvent;
c. extracting a tobacco extract from the tobacco derived solid to form a liquid tobacco extract and solvent mixture; and
d. providing a cartridge of an inhaler article and delivering the tobacco extract to the cartridge.
